# EUROPEAN PATENT APPLICATION

(11) **EP 4 834 286 A1**
(43) Date of publication of application: **04.11.2026**
(21) Application number: 24911466.1
(22) Date of filing: 27.12.2024
(51) Int. Cl.: A61F 2/24

(54) **IMPLANTABLE DEVICE AND MANUFACTURING METHOD THEREFOR**

(30) Priority: 29.12.2023 CN 202311855406
(71) Applicant: Biotyx Medical (Shenzhen) Co., Ltd., Shenzhen, Guangdong 518000 (CN)
(72) Inventor: ZHANG, Gui, Shenzhen, Guangdong 518000 (CN); ZHANG, Deyuan, Shenzhen, Guangdong 518000 (CN)
(74) Representative: Prinz & Partner mbB
(86) International application number: PCT/CN2024/143166
(87) International publication number: WO 2025/140546

(57) **Abstract**

The present application relates to the technical field of medical devices, and specifically, to an implantable device and a manufacturing method therefor. The implantable device includes a body and an adhesion portion, where the body includes a first subportion and a second subportion. The first subportion is configured to be apposed to a wall of a lumen after the implantable device is implanted into the lumen. The second subportion is connected to the first subportion and is at least partially suspended after the implantable device is implanted into the lumen. The adhesion portion is attached to the second subportion. The implantable device provided by the present application can improve overall safety of the device from multiple aspects.

## Description

### Technical Field

The present application relates to the technical field of medical devices, and specifically, to an implantable device and a manufacturing method therefor.

### Background Art

Heart valves are unidirectional valves located between atria and ventricles, or between ventricles and great arteries. Valvular heart disease is one of the most common cardiovascular diseases. Clinically, structural or functional abnormalities of one or more valves caused by rheumatic inflammation, degenerative changes, congenital malformations, ischemic necrosis, trauma, etc., can lead to valvular stenosis or insufficiency. For mild cases of valvular heart disease, symptoms can be alleviated with medication. In severe cases, valve repair may be performed, while for patients who are not suitable candidates for repair, prosthetic heart valve replacement is required.

Heart valve replacement refers to the replacement of a diseased native valve with a prosthetic mechanical heart valve made of synthetic materials or a bioprosthetic heart valve made of biological tissues. Since prosthetic heart valves have a limited lifespan and their dimensions are fixed, especially in children and adolescents, multiple replacements are required to accommodate the recipient's growth. Before implanting a new prosthetic heart valve, the previously implanted prosthetic heart valve needs to be explanted. However, due to tissue adhesions in the surgical area, the removal procedure via open-heart surgery is associated with significant difficulty and risk. Accordingly, stents of some prosthetic heart valves are made of bioresorbable materials, which gradually degrade in vivo. As a result, when a new prosthetic heart valve is implanted, the old one does not need to be removed, thereby greatly reducing the surgical risk.

Although the use of bioresorbable materials for manufacturing valve stents offers numerous advantages, some bioresorbable materials, such as magnesium alloys, corrode too quickly. This can lead to early structural incompleteness after device implantation, causing the stent to lose its intended supporting function for the tissue, ultimately resulting in device failure and severely compromising its safety and reliability. Additionally, the wall thickness of valve stents or scaffolds is generally on the order of hundreds of micrometers, which can easily form sharp edges that may cause tissue injury, thereby also affecting device safety to some extent. Furthermore, there is the potential for local stent fracture or the detachment of large degradation fragments, which could travel into downstream blood vessels and cause embolism. Moreover, due to the complexity and individual variability of human anatomy, the shape of prosthetic heart valve stents is often difficult to perfectly match the diverse anatomical structures of patients. After some prosthetic heart valves are implanted into the human body, variations in the degree of apposition between the stent and the luminal wall, as well as differences in blood flow velocity, can lead to significant heterogeneity in endothelialization. Sites with slower endothelialization may become prone to thrombus formation. In summary, currently available implantable devices have relatively low safety profiles.

### Summary

To solve the above technical problems, the technical solution of the present application provides an implantable device which can improve overall safety of the device.

In a first aspect, the present application provides an implantable device, which includes: a body, where the body includes a first subportion and a second subportion, the first subportion is configured to be apposed to the wall of a lumen after the implantable device is implanted into the lumen, and the second subportion is connected to the first subportion and is at least partially suspended after the implantable device is implanted into the lumen; and
an adhesion portion, where the adhesion portion is attached to the second subportion.

In some embodiments, the shape of the adhesion portion is configured to conform to changes in the shape of the second subportion.

In some embodiments, the implantable device further includes a limiting member, where one end of the limiting member is connected to the first subportion, and the other end of the limiting member is connected to the second subportion and/or the adhesion portion.

In some embodiments, at least two limiting members are disposed, and the limiting members are respectively connected to different positions of the second subportion and/or the adhesion portion.

In some embodiments, the second subportion includes at least one strut, the adhesion portion includes at least one winding thread, and the at least one winding thread is wound around the strut in a plurality of turns to form the adhesion portion.

In some embodiments, the filament width or diameter of the winding thread is 50-700 µm.

In some embodiments, a spacing between adjacent turns of the winding thread is 0-0.5 mm.

In some embodiments, each winding thread includes a plurality of fibers, and the fibers have diameters ranging from 5 µm to 25 µm, which may be uniform or varied.

In some embodiments, the winding thread is wound around the strut in a manner selected from one or any combination of the following: overlapping winding; flat winding; multi-layered winding; and variable-density winding.

In some embodiments, the included angle between each turn of the winding thread and the strut axis is less than 90°.

In some embodiments, the included angle between each turn of the winding thread and the strut axis varies along the axis.

In some embodiments, the winding thread forms a plurality of binding knots on the strut, and the binding knots are disposed adjacently or at intervals.

In some embodiments, the adhesion portion further includes a traction layer, the traction layer is disposed along the length direction of the strut of the second subportion, the winding thread and the traction layer form a plurality of intersection points, and at each intersection point, the winding thread and the traction layer are connected or spaced apart to form an integrated structure.

In some embodiments, the adhesion portion includes a base layer and a plurality of fibers extending from the base layer, the base layer is wrapped around the second subportion, and the fiber distribution density is 10-500 fibers/mm².

In some embodiments, the adhesion portion has a thickness of 10-750 µm. Further, the adhesion portion has a thickness of 10-150 µm.

In some embodiments, the second subportion includes a tip, and the adhesion portion has a thickness of 50-150 µm at the tip.

In some embodiments, the second subportion includes a fracture-prone region, and the adhesion portion has a thickness of 20-100 µm in the fracture-prone region.

In some embodiments, the adhesion portion is provided with a plurality of pores, each pore having a pore diameter of less than 500 µm.

In some embodiments, the adhesion portion includes a first density region and a second density region disposed adjacent to one another, the first density region is provided with a plurality of pores with a diameter ranging from 10 µm to 100 µm, and the second density region is provided with a plurality of pores with a diameter ranging from 150 µm to 500 µm.

In some embodiments, the body or the second subportion is biodegradable, and the degradation time of the second subportion is longer than the endothelialization time of the second subportion.

In some embodiments, the adhesion portion is non-degradable, or the degradation time of the adhesion portion is longer than the endothelialization time of the second subportion.

In some embodiments, the surface of the adhesion portion has wavy undulations with a peak-to-valley height of 0.03-0.7 mm.

In some embodiments, the adhesion portion has a permeability coefficient ranging from 1×10⁻¹³ to 1×10⁻³ cm/s.

In some embodiments, the material of the adhesion portion exhibits a procoagulation activity of 10%-90%.

In some embodiments, both the adhesion portion and the first subportion have negative surface potential polarity, and the absolute value of the surface potential of the adhesion portion is less than that of the first subportion.

In some embodiments, the body further includes a plurality of hollowed-out portions, the hollowed-out portions extend through an inner surface and an outer surface of the body, a flow channel is formed in the axial direction of the body, the second subportion is adjacent to at least one of the hollowed-out portions, and the adhesion portion is attached to the second subportion in a manner that the second subportion is suspended and/or a fluid within the lumen can flow out from the flow channel through the hollowed-out portion adjacent to the second subportion.

In some embodiments, the body is of a hollowed-out tubular structure, and the caliber of the first subportion is smaller than the caliber of the second subportion.

In some embodiments, the length of the first subportion is greater than the length of the second subportion.

In some embodiments, the implantable device further includes a leaflet assembly and a skirt portion, and the leaflet assembly is fixed to the inner side of the first subportion.

In some embodiments, the skirt portion includes an inner skirt and/or an outer skirt, where the inner skirt is configured to connect the leaflet assembly to the body, and the outer skirt covers the outer side of the first subportion.

In another aspect, the present application provides a manufacturing method for an implantable device, which includes:
selecting a base material for preparing the implantable device;
processing the base material to form a body of the implantable device, where the body includes a first subportion configured to be apposed to the wall of a lumen after the implantable device is implanted into the lumen, and a second subportion which is at least partially suspended after the implantable device is implanted into the lumen; and
forming an adhesion portion on the second subportion.

When the implantable device provided by the present application is implanted into a living body, the first subportion of the body, due to contact with living tissue, undergoes relatively rapid tissue endothelialization and becomes fixed within the tissue. In contrast, the second subportion, which is suspended without contacting the living tissue, is provided with the adhesion portion. The adhesion portion confines any detached fragments that may be generated from degradation, fracture, or other causes of the second subportion prior to endothelialization, thereby preventing the detached fragments from migrating within the living body and causing thrombosis, avoiding tissue injury caused by sharp edges of the body itself, and reducing the risk of puncturing the tissue or a delivery balloon during device delivery. Furthermore, the adhesion portion promotes endothelialization of the suspended second subportion, enhances the uniformity of endothelialization across different parts of the body, and regulates corrosion and degradation of the second subportion to improve degradation characteristics of the implantable device. As a result, the implantable device can exhibit delayed corrosion in an early post-implantation stage to provide sufficient structural support to the lumen, and then undergo rapid degradation in a later stage to relieve unnecessary constraints on the lumen. This improves the overall safety of the implantable device during both the implantation procedure and various post-implantation stages.

### Brief Description of the Drawings

In order to describe the technical solutions in the embodiments of the present application more clearly, the drawings required to be used in the embodiments will be simply introduced below. It is apparent that the drawings described below are only some embodiments of the present application. Other drawings may further be obtained by those of ordinary skill in the art according to these drawings without creative work.
FIG. 1 is a schematic structural diagram of an implantable device provided by an embodiment of the present application;
FIG. 2 is a schematic state diagram of an implantable device after implantation according to an embodiment of the present application;
FIG. 3 is an enlarged schematic diagram of part A of the implantable device shown in FIG. 1;
FIG. 4 is an exemplary schematic structural diagram of an adhesion portion of the implantable device according to the present application;
FIG. 5 is another exemplary schematic structural diagram of an adhesion portion of the implantable device according to the present application;
FIG. 6 is another exemplary schematic structural diagram of an adhesion portion of the implantable device according to the present application;
FIG. 7 is another exemplary schematic structural diagram of an adhesion portion of the implantable device according to the present application;
FIG. 8 to FIG. 11 are schematic diagrams of an implantable device according to some other embodiments of the present application;
FIG. 12 is a flowchart of a manufacturing method for an implantable device according to an embodiment of the present application;
FIG. 13 is a partial scanning electron microscope (SEM) image of an adhesion portion of an implantable device according to Example 1 of the present application;
FIG. 14 is a view showing an endothelialization effect of the implantable device according to Example 1 of the present application;
FIG. 15 is a partial SEM image of an adhesion portion shown in FIG. 14 at a higher magnification;
FIG. 16 is a partial SEM image of an implantable device in Example 1 of the present application after a period of implantation; and
FIG. 17 is an SEM image of part E shown in FIG. 16 at a higher magnification.

### Detailed Description

The exemplary implementations of the present application will be described in detail with reference to the drawings. Although the drawings show the exemplary implementations of the present application, it should be understood that the present application may be embodied in various forms and should not be limited by the implementations set forth herein. Rather, these implementations are provided such that the present application will be thoroughly understood, and the scope of the present application will be fully conveyed to those skilled in the art.

It should be noted that in the present application, a symbol "/", if any, means "or". For example, "needed/applicable in vivo" means needed in vivo or applicable in vivo.

Unless otherwise defined, all technical and scientific terms used herein have the same meanings as commonly understood by those of ordinary skill in the art to which the present application belongs. In case of conflict, this document, including definitions, shall prevail. Preferred methods and materials are described below, although methods and materials similar or equivalent to those described herein can also be used in the implementation or testing of the present application. The materials, methods, and implementations disclosed herein are illustrative only and are not intended to be limiting.

The term "about" or "approximately" as used with respect to an amount include variations in the recited amount that are equivalent to the recited amount, e.g., an amount that is not substantially different from the recited amount for the intended purpose or function.

For ease of description, spatial relative relationship terms may be used in the text to describe a relationship between one element or feature and another element or feature as shown in the figure. These relative relationship terms include, for example, "interior", "exterior", "inner side", "outer side", "below", "under", "above", "over", and the like. Such spatially relative relationship terms are meant to include different orientations of a device in use or operation other than orientations depicted in the figures. For example, if the device in the figures is overturned, an element described as "under" or "below" other elements or features will then be oriented as "above" or "over" other elements or features. Accordingly, the example term "below" may include both an upper orientation and a lower orientation. The device may otherwise be oriented (rotated 90 degrees or in other directions) and a spatial relative relationship descriptor used herein is interpreted accordingly.

It should be understood that the terminology used herein is for the purpose of describing specific implementations only and is not intended to be limiting. As used herein, the singular forms "a", "an", and "the" may be intended to include the plural forms as well, unless the context clearly indicates otherwise. The terms "comprises", "comprising", "including", and "having" are inclusive and therefore specify the presence of stated features, steps, operations, elements, and/or components, but do not preclude the presence or addition of one or more other features, steps, operations, elements, components, and/or combinations thereof. The method steps, processes, and operations described herein are not to be construed as necessarily requiring their performance in the particular order described or illustrated, unless specifically identified as an order of performance. It should also be understood that additional or alternative steps may be employed.

The present application provides an implantable device. The implantable device may be a related stent implanted inside a human body to achieve a supporting function, particularly various stents, prostheses, filters, and the like suitable for implantation in various lumens of the human body. For example, it may be of different types such as a vascular stent, a ureteral stent, a biliary stent, an esophageal stent, an airway stent, a renal artery stent, a heart valve, an occluder, a vascular puncture site closure device, or a vena cava filter. Depending on the site of a lesion, a corresponding type of implantable device is used to achieve treatment. In the implementation solutions of the present application, for ease of explanation, the implantable device is described by taking a prosthetic heart valve as an example, and this is not intended to limit the scope of its functions.

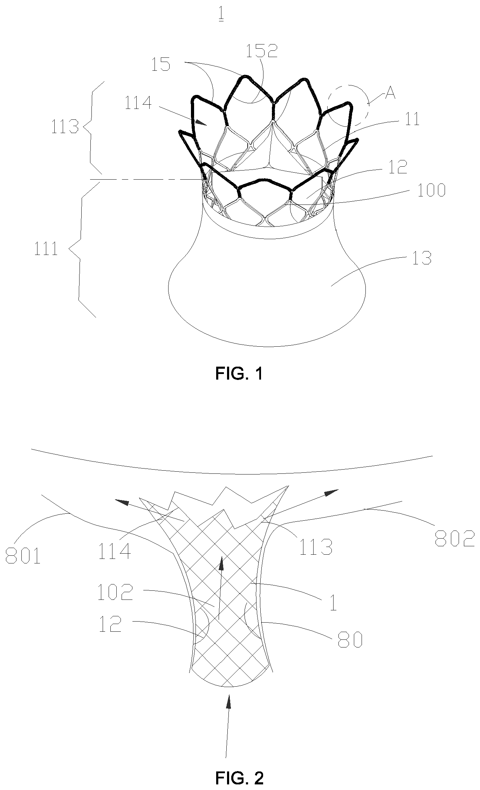

Referring to FIG. 1 to FIG. 3, the implantable device 1 provided by the present application includes a body 11 and an adhesion portion 15. The body 11 is configured to have a predefined shape as required. A flow channel 102 is typically formed inside the body 11 to allow normal blood flow through the internal flow channel 102. The flow channel 102 is typically formed along the axial direction of the body 11. Referring to FIG. 2, an arrow in a generally vertically upward direction indicates blood flow passing through the flow channel 102 of the implantable device 1 within a lumen. Specifically, the implantable device 1 may be a prosthetic heart valve, and a leaflet assembly 12 is opened to allow antegrade blood flow to exit from the flow channel 102. A sectional profile of the body 11 may be configured to have different shapes, such as a circle, an oval, a pentagon, a hexagon, and other suitable shapes, according to different application requirements. The body 11 is configured to support a living body at an implantation site and/or configured to be fixed to a living body, to achieve positioning and connection between the entire implantable device 1 and the living body. After the body 11 is implanted into the living body, at least part of the body 11 is apposed to the living body to provide support. For example, when the body is implanted at the site of a stenotic lesion in a blood vessel, the body 11 is apposed to the blood vessel, to provide support for the stenotic blood vessel, such that the cross-sectional area of the channel for blood flow in the blood vessel is enlarged and the blood vessel is no longer stenotic.

The body 11 includes a first subportion 111 and a second subportion 113. The first subportion 111 is configured to be apposed to the wall of a lumen after the implantable device 1 is implanted into the lumen. The second subportion 113 is connected to the first subportion 111 and is at least partially suspended after the implantable device 1 is implanted into the lumen. The body 11 is of a hollowed-out tubular structure, and includes a plurality of hollowed-out portions 114. The hollowed-out portions 114 extend through an inner surface and an outer surface of the body 11. The adhesion portion 15 is attached to the second subportion 113. After the implantable device 1 is implanted into a living body, the first subportion 111 of the body 11, due to contact with living tissue, undergoes relatively rapid tissue endothelialization and becomes fixed within the tissue. In contrast, the second subportion 113, which is suspended without contacting the living tissue, is provided with the adhesion portion 15, such that the adhesion portion 15 confines any detached fragments that may result from degradation, fracture, or other causes of the second subportion 113 prior to endothelialization, thereby preventing the detached fragments from migrating within the living body and causing thrombosis, avoiding tissue injury caused by sharp edges of the body 11, and reducing the risk of puncturing the tissue or a delivery balloon during device delivery. Furthermore, the adhesion portion 15 may also promote endothelialization of the suspended second subportion 113, enhance the uniformity of endothelialization across different parts of the body 11, and regulate corrosion and degradation of the second subportion 113 to moderately delay or accelerate the degradation rate of the body 11, such that the implantable device 1 can maintain its structural integrity for a desired period of time to sufficiently and effectively support the lumen, and can rapidly complete degradation beyond the desired period of time, thereby ensuring the effectiveness of the implantable device 1 within the desired period of time and relieving unnecessary constraints on the lumen as soon as possible after the implantable device 1 achieves its functions. This improves the overall safety of the implantable device during both the implantation procedure and various post-implantation stages.

In some feasible embodiments, the caliber of the first subportion 111 is smaller than the caliber of the second subportion 113. Still further, the second subportion 113 may be located at an end of the body 11, such as at an outflow end of the implantable device 1. Of course, it may also be located at an inflow end, or may be located at various positions such as a bifurcated vessel, a vessel ostium, or a center of a lumen after implantation. The second subportion 113 with a larger caliber can stabilize the implantable device 1 at a target site within the living body, thereby preventing displacement of the implantable device 1 caused by various reasons (such as cardiac contraction and relaxation), which would otherwise fail to provide stable support for the target site. This reduces the overall risk associated with implanting the implantable device 1 in complex anatomical structures.

In some feasible embodiments, the adhesion portion 15 is attached to the second subportion 113 in a manner that fluid within a lumen can flow out from the flow channel 102 through the hollowed-out portion 114 adjacent to the second subportion 113. In an example, as shown in FIG. 1 and FIG. 2, the body 11 of the implantable device 1 is a valve frame of a heart valve, which is implanted in a pulmonary artery 80. The first subportion 111 is apposed to an inner wall of the pulmonary artery 80, while the second subportion 113 is proximate to the outflow end and is suspended at a confluence/junction of the pulmonary artery 80 and a pulmonary artery branch. The hollowed-out portions 114 extending through the inner surface and the outer surface of the body allow blood flowing along the flow channel 102 to exit from the hollowed-out portions 114 on both left and right sides and flow into a right pulmonary artery 801 and a left pulmonary artery 802, respectively (as indicated by an arrow in FIG. 2). This maintains smooth blood flow, and reduces blood flow abnormalities caused by the implanted device, thereby improving patency of distal pulmonary artery branches, and enhancing the safety of the implantable device 1 implanted in complex anatomical structures such as a right ventricular outflow tract. In an example, the length of the first subportion 111 is greater than the length of the second subportion 113. In another example, the adhesion portion 15 is attached to the second subportion 113 in a manner that the second subportion 113 is kept suspended, that is, the second subportion 113 and the adhesion portion 15 attached thereto are kept suspended together. Of course, in some other feasible embodiments, the adhesion portion 15 is attached to the second subportion 113 in a manner that the adhesion portion covers at least part of the hollowed-out portions 114. In an example, the adhesion portion 15 may be a film layer covering the surface of the second subportion and the hollowed-out portions 114, and the film layer may be specifically, for example, an electrospun layer.

In some feasible solutions, different parts of the implantable device exhibit substantially similar endothelialization times. The first subportion 111 of the body 11 is apposed to the wall of a lumen and undergoes relatively rapid tissue endothelialization, such that the first subportion 111 is covered by a generated endothelial layer at a relatively fast rate. The suspended second subportion 113 would otherwise exhibit delayed tissue endothelialization due to suspension or lack of contact with a living body. However, the second subportion 113 is provided with an adhesion portion 15 attached. The adhesion portion 15 can accelerate the capture of endothelial cells and endothelial progenitor cells from blood without compromising smooth blood flow within the lumen. Consequently, the suspended second subportion 113 (with the adhesion portion 15) and the apposed first subportion 111 of the implantable device 1 exhibit substantially similar endothelialization times, thereby improving the uniformity of the overall endothelialization process of the device, reducing the risk of thrombus formation, and enhancing the safety of the device.

It should be understood that substantially similar endothelialization times used herein refer to a condition in which, compared to the implantable device 1 lacking the aforementioned adhesion portion 15, the endothelialization time of the second subportion 113 provided with the adhesion portion 15 is closer to the endothelialization time of the first subportion 111, or the endothelialization rate of the suspended second subportion 113 is accelerated, such that the endothelialization time of this portion is reduced by more than 50%, or even by 80% to 95%. For example, under conditions where the body 11 has the same wall thickness, the endothelialization time of the first subportion 111 ranges from 7 days to 3 months, and endothelialization is typically achieved within about 1 month. In contrast, the second subportion 113 without the adhesion portion 15 fails to achieve endothelialization even after more than 1 year or even 2 years, whereas the second subportion 113 provided with the adhesion portion 15 exhibits the endothelialization time of approximately 7 days to 3 months. In another definition, substantially similar endothelialization times may refer to the condition in which, at a time point when endothelialization of the first subportion 111 is achieved, or at a predetermined empirical time point (e.g., 14 days, 1 month, 45 days, or 2 months), the degree of endothelial coverage at the second subportion 113 is substantially the same as that at the first subportion 111, with the difference in endothelial coverage between the two being within 10%. It should be understood that as used herein, the endothelialization time of the second subportion 113 refers to the time required to form an endothelial layer that covers the adhesion portion 15 and the corresponding second subportion 113. Specifically, the endothelialization time may refer to the time required to form an endothelial layer that encapsulates both the second subportion 113 and the adhesion portion 15 within a region of the second subportion 113 where the adhesion portion 15 is provided. The region where the adhesion portion is provided is defined by boundaries of the adhesion portion 15. The endothelialization time of the first subportion 111 refers to a time required to form an endothelial layer that covers the first subportion 111.

In some embodiments, the body 11 or the second subportion 113 is biodegradable. For example, the first subportion 111 and the second subportion 113 may be made of the same biodegradable material, or different biodegradable materials. The biodegradable materials may be, for example, iron, an iron-based alloy, magnesium, a magnesium-based alloy, zinc, a zinc-based alloy, or a bioresorbable polymer material. In some embodiments, during the gradual degradation process of the second subportion 113, the wall thickness and the strut width of the strut 100 become smaller. In an example, the adhesion portion 15 is attached to the strut 100 in a manner of wrapping around the strut, such that the adhesion portion may wrap large detached fragments generated from degradation of the strut 100 therein, and only ions generated from the degradation are allowed to seep out from the adhesion portion, thereby preventing the large detached fragments from falling into a blood vessel and causing thrombosis. Further, the shape of the adhesion portion 15 may be configured to conform to changes in the shape of the second subportion 113. Thus, during the degradation process of the second subportion 113, the connection between the adhesion portion 15 and the second subportion 113 remains relatively tight, and the detached fragments generated from the degradation of the second subportion 113 are less likely to separate from the adhesion portion 15. Various approaches exist for allowing the shape of the adhesion portion 15 to vary in accordance with the shape change of the second subportion 113. For example, the adhesion portion 15 may be elastic, and the adhesion portion 15 may elastically contract in response to a reduction in wall thickness and strut width of the second subportion 113. For another example, the adhesion portion 15 may be made of a flexible material, such that the adhesion portion 15 is prone to deformation.

In some embodiments, the degradation time of the second subportion 113 is longer than the endothelialization time of the second subportion 113. By attaching the adhesion portion 15 to the second subportion 113, the endothelialization process of the second subportion is accelerated, and an encapsulating endothelial layer is formed before degradation of the second subportion 113. This endothelial layer is configured to retain large detached fragments generated from degradation or fracture of the second subportion 113, thereby preventing such large detached fragments from bloodstream, further reducing the risk of thrombosis arising therefrom, and enhancing the safety and reliability of the device.

In an example, when the body 11 is a nitrided iron-based stent with a wall thickness of about 140 µm, and the second subportion 113 does not have the adhesion portion 15, degradation begins at about 3 months after implantation. The degradation extent (calculated by weight loss) is about 20% at 6 months, about 40% at 12 months, about 60% at 24 months, and more than 80% at 36 months. In another example, for a pure iron-based stent, the degradation extent (calculated by weight loss) is about 10% at 6 months, about 25% at 12 months, about 40% at 24 months, and about 50% at 36 months after implantation.

In some embodiments, the adhesion portion 15 is made of a biodegradable material. Further, the degradation time of the adhesion portion 15 is longer than the endothelialization time of the second subportion 113. For example, the adhesion portion 15 is formed by winding a poly-L-lactic acid (PLLA) suture line around the second subportion 113, and its degradation time ranges from several months to 2 years. In other examples, the adhesion portion 15 may also be made of other polymer materials, such as polyethylene (PE) and polycaprolactone (PCL), which are not enumerated one by one herein. The degradation time of the adhesion portion 15 is longer than the endothelialization time of the second subportion 113, which ensures that the adhesion portion 15 has sufficient time to capture endothelial cells and to allow formation of an endothelial layer before fracture of the second subportion 113. In an example, the adhesion portion 15 is made of a compliant material and can wrap around and cover any sharp edges and tips that may exist on the second subportion 113, thereby reducing damage to surrounding tissue or components of a delivery device, such as a balloon during implantation or after the device is deployed in place. In other examples, the adhesion portion 15 may also be made of a combination of a biodegradable material and a non-biodegradable material in a suitable ratio, or entirely made of a non-degradable material.

In some embodiments, different portions of the body 11, such as the first subportion 111 and the second subportion 113, may be integrally formed, or may be detachably connected, for example, by suturing, bonding, or snapping engagement. The adhesion portion 15 may be attached to the second subportion 113 by winding, coating, deposition, or wrapping.

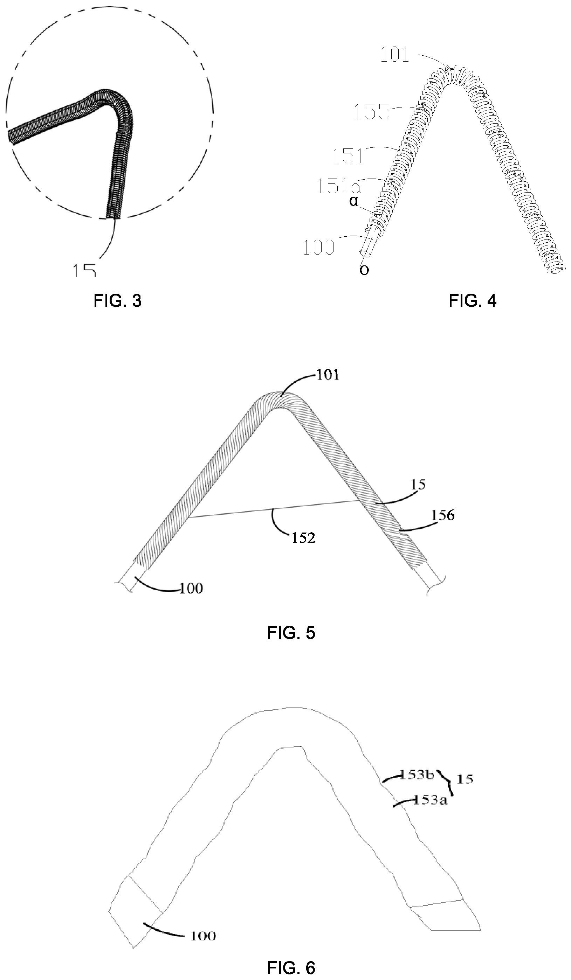

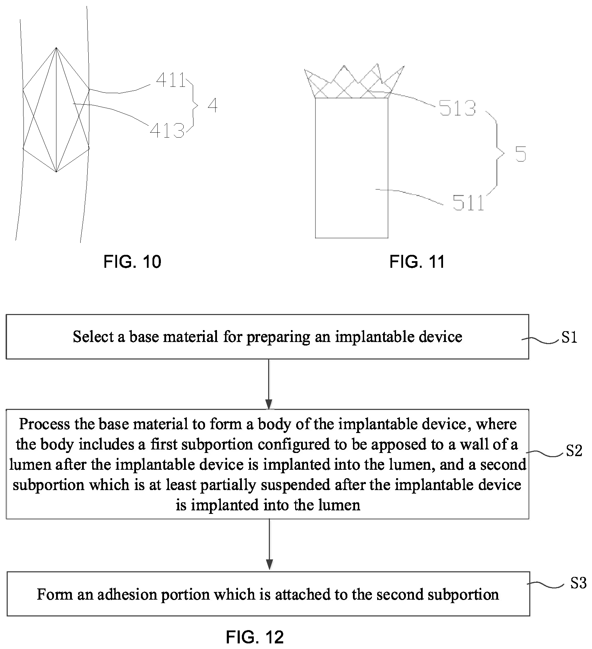

Referring again to FIG. 1 and FIG. 3, in some embodiments, the second subportion 113 and the first subportion 111 respectively include a plurality of struts 100, where a certain number of struts 100 may define a hollowed-out portion 114, that is, the second subportion 113 is adjacent to at least one hollowed-out portion 114. For example, in FIG. 1, the body 11 is provided with a plurality of hexagonal hollowed-out portions 114 distributed circumferentially at a position closest to an outflow end, and each side of each hexagonal hollowed-out portion 114 corresponds to and is adjacent to one strut 100. Four struts 100 closer to the outflow end are wrapped with the adhesion portion 15 and are at least partially suspended after implantation in a living body, and belong to the second subportion 113 of the body 11; while two struts 100 corresponding and adjacent to the other two sides are exposed and not covered by the adhesion portion 15, and belong to the first subportion 111 of the body 11. In this example, the strut 100 may be a stent rod or a support rod of a vascular stent or a valve frame. In some other examples, such as a vena cava filter in FIG. 10, the corresponding strut refers to a filter rod. Other examples are not enumerated one by one herein.

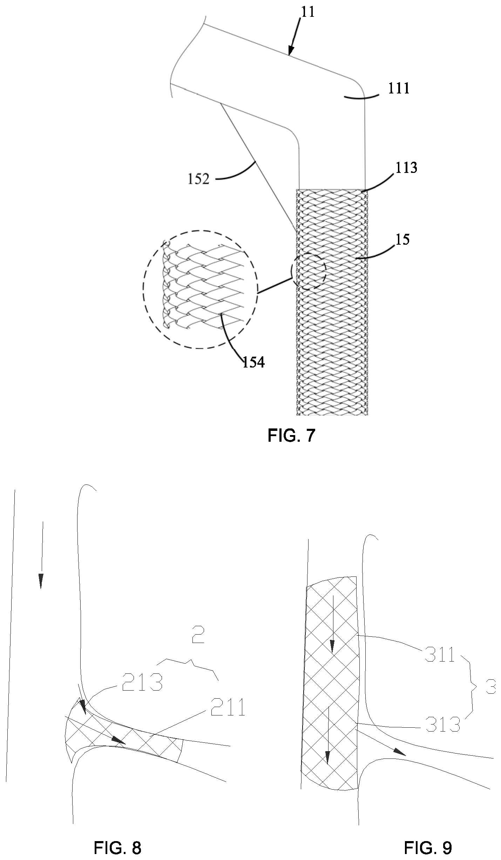

In some embodiments, the adhesion portion 15 covers the strut 100 along the length direction of the strut 100, such that the strut 100 can be wrapped in the circumferential and length directions and is not provided with any exposed portions. In another example, the adhesion portion 15 is attached to the strut by surrounding the strut 100 and allows the surface of the strut 100 to be partially exposed. In an example, struts 100 suspended after the second subportion 113 is implanted into a living body may be wrapped to form a corresponding adhesion portion 15, while struts 100 apposed after the second subportion 113 is implanted into the living body may be surrounded to form a corresponding adhesion portion 15. In another example, after the second subportion 113 is implanted into a living body, the adhesion portion 15 attached to a suspended strut 100 and the adhesion portion 15 attached to an apposed strut 100 may have different surface morphologies, permeability coefficients, thicknesses, materials, and the like. Of course, these may also be the same. In embodiments of the present application, the proportion of the second subportion 113 in the body 11 is greater than 0%, the proportion of a suspended portion after the second subportion 113 is implanted into a living body in the second subportion 113 is greater than 0%, and specific proportions may vary according to different device types and implantation sites. Further, the second subportion 113 may account for 1-99% of a surface area of the body 11. Of course, the proportion may also be calculated in other manners. For example, the proportion may be calculated based on the length of the body 11 and the length of the second subportion 113. Further, the portion that is suspended but not apposed after the second subportion 113 is implanted into a living body may account for 0.1%-100% of the second subportion 113. In a specific example, as shown in FIG. 1, the second subportion 113 of the implantable device 1 accounts for 20% of a length of the body 11, and the suspended portion of the second subportion 113 after implantation accounts for 50% of a length of the second subportion 113. In another example, referring to FIG. 8, the second subportion 313 accounts for 10% of the length of the body, and the suspended portion of the second subportion 313 after implantation accounts for 85% of the length of the second subportion 313. In another example, referring to FIG. 10, the surface area of the second subportion 413 accounts for 99% of the surface area of the body, and the suspended portion of the second subportion 413 after implantation accounts for 100% of the surface area of the second subportion 413.

Referring to FIG. 4, in some embodiments, the adhesion portion 15 may be a winding layer, the winding layer includes at least one winding thread 151, and at least one winding thread 151 is wound around a strut 100 of the second subportion 111. In an example, each winding thread includes a plurality of fibers, such that each winding turn concurrently forms a plurality of wound fibers. Further, adjacent turns of the winding thread may be oriented at varying angles relative to the axis of the strut 100, e.g., deflected leftward or rightward, and may also overlap one another. This configuration serves a dual purpose: first, it resists displacement of the winding layer on the strut 100; and second, it allows the fibers of different turns to alternately cover the strut 100, thereby forming a generally three-dimensional wrapping structure. Interlaced gaps among the fibers define intricate microfluidic pathways, enabling the permeability coefficient of the adhesion portion 15 to be tailored within a therapeutically effective range for modulating degradation characteristics of the strut 100, ultimately achieving regulation of the degradation rate of the strut 100. In addition, the adhesion portion 15 exhibits a relatively balanced roughness across different regions thereof, with reduced variation in peak-to-valley heights of wavy undulations. This promotes uniform film formation in all regions of the adhesion portion 15. Further, the filament width or diameter of the winding thread is 50-700 µm. The fibers have the same or different diameters ranging from 5 µm to 25 µm, preferably 9 µm to 16 µm. Specifically, the diameter may be, for example, 9 µm, 10 µm, 11 µm, 12 µm, 13 µm, 14 µm, 15 µm, or 16 µm, etc. This facilitates the formation of a peak-to-valley height ranging from 0.03 mm to 0.7 mm in different regions on the surface of the winding layer, thereby avoiding the formation of a relatively smooth local surface due to an excessively small local peak-to-valley height. Consequently, endothelial cells can rapidly and uniformly adhere to the adhesion portion 15, enabling the second subportion 113 to complete endothelialization quickly. It should be understood that a winding thread 151 may be formed by twisting a plurality of fibers into a single strand or interweaving them, or may be formed by arranging a plurality of fibers in parallel into a relatively loose bundle.

In some embodiment solutions of the present application, the thickness of the adhesion portion 15 may range from 10 µm to 750 µm. This thickness range is conducive to forming an undulating surface profile with a certain peak-to-valley height on the surface of the adhesion portion 15, thereby facilitating endothelialization and also preventing the adhesion portion 15 from being damaged, which would otherwise compromise the intended attachment configuration on the second subportion 113. Specifically, the thickness of the adhesion portion 15 may be 10-200 µm, 50-250 µm, 100-300 µm, 150-400 µm, 200-500 µm, 350-600 µm, or 400-700 µm, etc. Further, the thickness of the adhesion portion 15 may be 10-150 µm, which, on the basis of promoting endothelialization, can also avoid an excessive increase in the delivery profile to ensure ease of delivery, and can also avoid material waste. Specifically, the thickness of the adhesion portion 15 may include, but is not limited to 12-100 µm, 15-100 µm, 20-90 µm, 20-80 µm, 30-80 µm, 30-75 µm, 40-120 µm, 50-80 µm, 30-150 µm, or 50-130 µm, etc.

In some possible embodiment solutions, the thickness of the adhesion portion 15 may be configured to vary depending on different portions. For example, the thickness may be set greater at a portion where degradation is relatively more likely to occur than at another portion where the degradation rate is relatively lower. For another example, the thickness of the adhesion portion 15 may be configured to be relatively thick in the strut width direction of the strut 100, and relatively thin in the wall thickness direction of the strut. In this way, the adhesion portion 15 can reduce the impact on the delivery profile while maintaining a relatively good effect of promoting endothelialization of the suspended strut. For example, after the adhesion portion 15 is attached to a strut 100 of the second subportion 113, the strut width of the strut 100 may increase by 10-70 µm, and the wall thickness of the strut 100 may increase by 50-100 µm. Of course, in some other examples, the thickness of the adhesion portion 15 in the strut width direction of the strut 100 may also be less than or equal to the thickness of the adhesion portion 15 in the wall thickness direction of the strut 100.

In some embodiments, the strut 100 may include a tip 101, which may also be understood as a connection portion between two struts 100. The thickness of the adhesion portion 15 at the tip 101 may be relatively greater. In an example, the thickness of the adhesion portion 15 at the tip may be 50-150 µm. Further, the thickness of the adhesion portion 15 at the tip may be 60-100 µm, 50-90 µm, 60-80 µm, 60-70 µm, 60-90 µm, or 55-85 µm, etc. The adhesion portion 15 is configured to be relatively thicker at the tip 101, to further ensure that the adhesion portion 15 achieves better coverage at the tip 101, thereby enabling a more balanced endothelialization process between the struts 100 and connection portions thereof on the second subportion 113.

In some possible solutions, the second subportion 113 may include a fracture-prone region, and the adhesion portion 15 may have a greater thickness in the fracture-prone region, to reduce the likelihood of breakage of the fracture-prone region, such that the second subportion 113 has a more stable shape before endothelialization of the first subportion 111, thereby stabilizing the implantable device 1. The thickness of the adhesion portion 15 in the fracture-prone region may range from 20 µm to 100 µm, and a delivery profile can be taken into consideration.

The aforementioned fracture-prone region may be a portion of the strut 100 which is provided with an opening or a gap, a portion with a smaller strut width and wall thickness, or a portion with a greater stretching extent (such as a bend or a corner). It is not limited thereto; and any portion of the strut 100 that is more prone to fracture during the degradation process falls within the protection scope of the present application.

In some embodiments, the winding layer is wound around the strut in a manner selected from one or any combination of the following: overlapping winding; flat winding; multi-layered winding; and variable-density winding.

Referring again to FIG. 4, in an example, the winding thread 151 of the winding layer may be disposed in a winding manner on the strut 100 of the second subportion 113. The cross section of the winding thread 151 may be rectangular, circular, olive-shaped, star-shaped, polygonal, or the like. The width of the winding thread 151 may be more than, equal to, or less than the thickness of the winding thread 151. The winding thread in FIG. 4 adopts a single-layer flat-winding manner. Each turn of the winding thread is laid flat along the length direction of the strut 100. The spacing between adjacent turns may range from 0 mm to 0.5 mm. When the spacing exceeds 0.5 mm, a relatively large portion of the surface of the strut 100 remains exposed, which is unfavorable for wrapping and retaining fragments generated by corrosion of the strut 100. Moreover, it also takes more time for endothelial cells to adhere to the winding thread portion and gradually form a continuous endothelial layer.

The winding thread 151 may be disposed to circumferentially wind around the strut 100, and an included angle α between each turn of the winding thread 151 and the axis O of the strut 100 may be less than 90°. During the degradation process of the strut 100, the strut width and wall thickness of the strut 100 decrease gradually, the gap between the winding thread 151 and the strut 100 increases gradually. By setting the included angle α between each turn of the winding thread 151 and the axis of the strut 100 to be less than 90°, the displacement of the winding thread 151 in the axial direction of the strut 100 is relatively small. This facilitates the maintenance of relative positions of the winding thread 151 and the strut 100, and avoids the formation of an excessively large gap between adjacent turns due to displacement, thereby promoting uniform adhesion and growth of endothelial cells on the winding layer. Further, the included angle α between each turn of the winding thread 151 and the axis of the strut 100 may be 70°-89° or 80°-85°. This can not only reduce the likelihood of displacement, but also make the axial span of each turn relatively small, thereby facilitating the winding process. In another example, included angles α between each turn of the winding thread and the axis O may vary along the axis.

In a possible solution, the adhesion portion 15 may also further include a traction layer 155, the traction layer 155 is disposed along the length direction of the strut 100, the winding thread 151 wound around the strut 100 and the traction layer 155 have a plurality of intersection points, the intersection points are disposed at intervals of several turns, and a binding knot 151a is formed at each intersection point. At each intersection point, the winding thread 151 and the traction layer 155 are connected or spaced apart to form an integrated structure. Specifically, the traction layer 155 may be another winding layer located beneath the winding layer and having a sparser winding density, and the axial span between adjacent turns of the traction layer 155 is relatively larger. The traction layer 155 may also be configured in a linear shape with a substantially fixed length. In this manner, the winding thread 151 is connected to the traction layer 155, such that the traction layer 155 constrains both a length change of the winding thread 151 and the inter-turn spacing of adjacent turns. This prevents elongation of the winding thread 151 or localized displacement of the winding thread from the strut 100 after fracture or degradation of the strut 100. The traction layer 155 may also be coated, bonded, and sutured to the strut 100. The traction layer 155 may also include at least one of a connecting mesh, a connecting film, or a connecting coating. The traction layer 155 may be coated to the strut 100 in at least one manner of electrospinning, dip coating, drop coating, spray coating, or brush coating. When the traction layer 155 is a connecting mesh, the connecting mesh includes meshes; and when the traction layer 155 is the connecting film or the connecting coating, the connecting film or the connecting coating may be provided with holes.

In another possible implementation solution, the winding thread 151 itself may also include a plurality of binding knots, and the binding knots are disposed adjacently or at intervals. For example, the binding knots may be spaced apart by several turns. The binding knots can enhance the adhesion between the winding layer and the strut 100, making the adhesion portion 151 less likely to unravel from the strut 100. In addition, the binding knots can also increase the surface roughness of the adhesion portion 15, which is beneficial for promoting tissue endothelialization.

Optionally, the interval between two adjacent binding knots 151a may be 0.1-2 mm. When the interval between two adjacent binding knots 151a is less than 0.1 mm, the number of binding knots 151a is relatively great, thereby increasing the preparation difficulty and the delivery profile of the implantable device 1. When the interval between two adjacent binding knots 151a is more than 2 mm, the connecting effect of the winding thread 151 and the strut 100 is poor. Therefore, when the interval between two adjacent binding knots 151a is configured within the above value range, comprehensive performance is excellent.

Besides, the winding layer may also be braided using methods such as random braiding, square knot braiding, single square knot braiding, figure-eight braiding, cross knot braiding, lark's head knot braiding, double connection braiding, and single-line double connection braiding. Different braiding methods can differentiate a surface structure of the adhesion portion, thereby meeting requirements of different products or application scenarios.

Referring to FIG. 5, in an example, the adhesion portion 15 is a spun fiber layer, which can uniformly cover the strut 100 and the tip 101 of the second subportion 113. The spun fiber layer may be formed by winding a plurality of spun fibers at intervals around the strut 100, and a microgap 156 is formed between adjacent spun fibers. The width of the microgap 156 is less than 500 µm. Further, the width of the microgap 156 may include, but is not limited to 5-80 µm, 10-50 µm, 20-40 µm, 20-100 µm, 150-250 µm, 50-250 µm, 20-200 µm, 30-180 µm, or 100-300 µm, etc. Although the figure illustrates a single-layer structure of the spun fiber layer as an example, the present application is not limited thereto, and a multilayer structure may also be employed.

Referring to FIG. 6, in another example, the adhesion portion 15 may include a base layer 153a and a plurality of fibers 153b extending from the base layer 153a, the base layer 153a is tightly positioned over and around the strut 100 of the second subportion 113, and the distribution density of the fibers 153b is 10-500 fibers/mm².Thus, endothelial cells may adhere to and grow on the adhesion portion 15 more rapidly and uniformly, thereby achieving balanced endothelialization. Among the plurality of fibers 153b, the orientation, length, and thickness of different fibers 153b may be the same or different. The fibers 153b may have a curled shape, which can further facilitate the adhesion of endothelial cells. Of course, the fibers 153b may also be in a short and straight form. One end of the fiber 153b may be connected to the base layer 153a, and the other end is a free end; and two ends of the fiber 153b may be both connected to the base layer 153a. The fiber 153b may be a natural fiber, or a synthetic fiber.

FIG. 7 is a schematic structural diagram of an implantable device in another embodiment. In an example, the adhesion portion 15 is a woven mesh layer attached to the second subportion 113, and the woven mesh layer is provided with a plurality of micropores 154. In some other embodiments, the adhesion portion 15 may also be a porous film. The shape of pores is not limited to a circle, a square, a rectangle, a rhombus, a triangle, or any other shape. In some examples, the pore diameter of a single pore is less than 500 µm. Further, the diameter of the single pore may include, but is not limited to 5-80 µm, 10-50 µm, 20-40 µm, 20-100 µm, 150-250 µm, 50-250 µm, 20-200 µm, 30-180 µm, or 100-300 µm, etc.

In an example in FIG. 7, the pores have the same size and are uniformly distributed; and in some other embodiments, the adhesion portion 15 may include a first density region and a second density region disposed adjacent to one another, and the diameter of the pores in the second density region is greater than the diameter of the pores in the first density region. Because contact with blood (fluid) accelerates the degradation of the strut 100, a portion of the strut 100 having a relatively larger contact area with blood degrades at a higher rate. Through the above configuration, the adhesion portion 15 may have a certain regulation effect on the degradation of the strut 100, that is, through the above configuration, a region of the strut 100 which is fractured first may be set.

Further, the diameter of a single pore in the first density region may be 10-100 µm, and the diameter of a single pore in the second density region may be 150-500 µm. Thus, the degradation of the strut 100 can be regulated by configuring different diameters of the pores.

In addition to the winding layer, the base layer with fibers, the porous film, the woven mesh layer, and the spun fiber layer, the structure of the adhesion portion 15 may also be in the form of other permeable membranes, permeable coatings, and the like, and may also be a combination of different structures.

In some possible embodiments, the surface of the adhesion portion 15 of the foregoing different structures has wavy undulations with a peak-to-valley height of 0.03-0.7 mm. The adhesion portion 15 with this surface feature can promote endothelialization, such that the endothelialization time of the second subportion 113 is within 3 months. When the peak-to-valley height is less than 0.03 mm, the wavy undulations on the surface are not significant. As a result, endothelial cells passing by cannot effectively anchor to the surface of the adhesion portion 15, inhibiting endothelial cell proliferation on the adhesion portion 15. Prolonged failure to achieve endothelial coverage may lead to platelet adhesion and aggregation, as well as inflammatory cell infiltration, thereby forming thrombi on the strut. This not only impairs blood flow, but also poses a risk of embolism upon thrombus dislodgement. However, according to the implantable device 1 in the present application, the adhesion portion 15 which promotes reduction of the endothelialization time to within 3 months is attached to the suspended second subportion 113. This correspondingly reduces thrombus formation on the device and enhances the safety of the device. When the peak-to-valley height is more than 0.7 mm, the wavy undulations on the surface are excessively significant, and the thickness or local thickness of the corresponding adhesion portion 15 is excessively great, thereby readily inducing adsorption of platelets and fibrin from blood and leading to device thrombosis in a short period of time. In addition, an excessively great peak-to-valley height is also unfavorable for delivery of the implantable device. Further, the peak-to-valley height on the surface of the adhesion portion 15 may include, but is not limited to 0.05-0.2 mm, 0.06-0.3 mm, 0.08-0.5 mm, 0.1-0.4 mm, or 0.15-0.55 mm, etc.

In some possible embodiments, the adhesion portion 15 of the foregoing different structures exhibits a procoagulation activity of 10%-90%. In combination with the wavy undulations on the surface of the adhesion portion 15, this can better promote endothelialization of the suspended second subportion 113 and the adhesion portion 15, such that the endothelialization is achieved within about 1 month. Further, the adhesion portion 15 exhibits the procoagulation activity of 15%-80%, 15%-60%, 15%-40%, 20%-75%, 25%-50%, 30%-70%, or 30%-55%. Two samples are taken, and the two samples are blood from the same source. A prothrombin time PT is measured using one of the samples as an object. The adhesion portion 15 (specifically, a portion of the second subportion 113 and the adhesion portion 15 attached thereto may be excised) is added to the other sample, and a prothrombin time PT is measured. The procoagulation activity is calculated as (PT-PT)/PT×100%. When the procoagulation activity of the adhesion portion 15 is below 10%, it is unfavorable for the landing and growth of endothelial cells from flowing blood on the adhesion portion 15, such that the time required for endothelialization of this portion exceeds 3 months. In contrast, when the procoagulation activity is above 90%, the adhesion portion 15 may cause excessively rapid local coagulation on the strut 100, resulting in device thrombosis.

In some possible embodiments, both the adhesion portion 15 of the foregoing different structures and the first subportion 111 have negative surface potential polarity, and the absolute value of the surface potential of the adhesion portion 15 is less than that of the first subportion 111. The negative surface potential polarity of the body 11 can reduce the probability of thrombosis. Moreover, configuring the absolute value of the surface potential of the adhesion portion 15 to be less than the absolute value of the surface potential of the first subportion 111 can further optimize the endothelialization times of the suspended portion and the apposed portion of the body 11, thereby making the difference between the two even smaller.

In some possible embodiments, the adhesion portion 15 of the foregoing different structures is water-permeable, to allow the passage of substances such as water molecules and ions generated by degradation of the implantable device 1. In some embodiments, the permeability coefficient of the adhesion portion may range from 1×10⁻¹³ to 1×10⁻³ cm/s, and may be further preferably selected from ranges such as 1×10⁻¹¹-1×10⁻⁴ cm/s, 1×10⁻¹⁰-1×10⁻⁵ cm/s, 1×10⁻⁹-1×10⁻⁶ cm/s, and 1×10⁻⁸-1×10⁻⁷ cm/s. Within these parameter ranges, the adhesion portion 15 can optimally regulate the degradation time of the strut 100 covered thereby. Specifically, when the strut 100 is made of a material with a high degradation rate, such as a magnesium alloy, the adhesion portion 15 having the aforementioned permeability coefficient can modulate the degradation time to delay the degradation of the strut 100. This prevents the strut 100 from degrading prematurely and failing to provide luminal support or positioning of the implantable device 1 within an intended period of time, and also allows the strut 100 to rapidly complete degradation after the intended period of time, thereby relieving unnecessary constraints on the implantation site.

For example, studies have shown that a magnesium alloy vascular stent completely degrades within approximately 1-3 months after implantation in a living body (e.g., implantation in a coronary artery). It has been reported that the first-generation magnesium alloy stent from Biotronik, which includes 93% of magnesium and 7% of rare earth elements and has a wall thickness of 165 µm, is completely degraded into ions within 60 days after implantation in the absence of a drug coating. If the second subportion 113 of the implantable device 1 is made of a magnesium alloy material, the struts 100 are degraded within a relatively short period of time. Large detached fragments potentially generated from degradation may flow with bloodstream to distal ends, readily causing embolism in distal blood vessels. In addition, the first subportion 111 is prone to separation from the second subportion 113 and subsequent displacement. This may cause, for example, a prosthetic pulmonary valve frame to slip from a right ventricular outflow tract into a right ventricle, resulting in device failure. The device failure rate is approximately 20%, which significantly compromises the safety performance of the device. However, when the aforementioned adhesion portion 15 is attached to the strut 100, the degradation time of the strut 100 can be extended to more than 1 year. Further, when the permeability coefficient of the adhesion portion 15 is within a range of 1×10⁻¹³-1×10⁻⁹ cm/s, a weight loss rate of the adhesion portion at 1 month, 2 months, and 3 months after implantation can be controlled to be within 5%, 20%, and 35%, respectively. The weight loss at 3 months can even be controlled below 10%, while complete degradation can be achieved within 1-2 years after implantation. Because the weight loss rate of the strut 100 remains relatively low for an extended period during an initial post-implantation stage, the strut can maintain a favorable structural morphology within a desired period after device implantation. This prevents device failure due to displacement and can reduce the device failure rate from 20% to below 1%. In addition, the degradation of the strut 100 can be regulated to occur after the completion of endothelialization, such that the adhesion portion 15 on the strut 100 and an endothelial layer can work together to encapsulate and confine the degraded debris of the strut 100, thereby reducing the risk of embolism.

In another example, when the strut 100 is made of a material with a relatively low degradation rate, such as an iron-based material, the adhesion portion 15 having the aforementioned permeability coefficient can ensure that a fluid within a lumen permeates to the surface of the strut 100. Moreover, the adhesion portion 15 made of a polymeric material can maintain a relatively high concentration of acidic environment around the wrapped strut 100 (since degradation of the strut or the adhesion portion itself generates acidic substances), thereby preventing the acid concentration from being diluted by blood flow. This can accelerate corrosion of the iron-based stent to a certain extent, such that the weight loss rate of the strut 100 is controlled to be below 10% at 3 months after implantation, while the degradation rate increases to over 20% at 6 months, and reaches 45%-55% at 12 months, and the strut can be completely degraded within approximately 2-3 years. At this point, the implantable device 1 has already completed the repair or support function at the implantation site. For instance, a leaflet assembly 12 has formed a secure connection with an endothelial layer of a blood vessel. Degradation of the body 11 relieves unnecessary constraints on the lumen, allowing the leaflet assembly to accommodate patient growth without hindering a secondary percutaneous valve-in-valve intervention for a larger prosthesis. Furthermore, this approach imposes no psychological burden on the patient and is suitable for patients of all ages. It should be understood that as the adhesion portion 15 gradually degrades in the living body, its permeability coefficient may undergo certain changes.

According to the implantable device 1 of an implementation of the present application, by regulating the corrosion and degradation of the second subportion to which the adhesion portion 15 is attached, the adhesion portion improves degradation characteristics of the body 11, which is made of bioresorbable materials that originally exhibit different degradation rates. This ensures that the device provides adequate structural support for tissue during an early post-implantation stage, and also allows the body to be degraded as soon as possible after the device achieves a therapeutic or repair purpose and tissue support is no longer required. Consequently, unnecessary constraint on the repaired or healthy lumen is avoided, complications are reduced, and other treatment or device implantation at the site is less likely to be performed. This enhances the overall safety of the implantable device 1 made of various materials during both initial and long-term post-implantation stages.

In another example of an embodiment of the present application, referring again to FIG. 1, FIG. 5, and FIG. 7, in some possible embodiments of the present application, the implantable device 1 further includes a limiting member 152. One end of the limiting member 152 is connected to the adhesion portion 15, and the other end is connected to different positions of the adhesion portion 15, or connected to the body 11. Specifically, the other end may be connected to the second subportion 113 or the first subportion 111. Specifically, the limiting member 152 may have a linear or strip-shaped structure, for example, a plurality of suspension-line structure shown in FIG. 1. Alternatively, the limiting member may also have other structures such as a mesh, a sheet, and a membrane. The limiting member 152 can further enhance a limiting effect on the second subportion 113 after a structure change of the second subportion after implantation. This prevents the second subportion 113 from escaping from the adhesion portion 15, or from detaching together with the adhesion portion 15 from the body, due to degradation or fracture. Such escape or detachment would otherwise cause the second subportion 113 to lose its positioning effect on the first subportion 111, leading to displacement or deviation of the first subportion from an implantation site and consequent failure of the implantable device. Fractured fragments that become dislodged and migrate to other sites can cause embolism or impair functions of normal organs. For example, after fracture of a pulmonary artery stent, fragments may travel to a contralateral pulmonary artery, or may migrate into a right ventricle where they can interfere with tricuspid valve closure. They may also travel to downstream vessels and serve as a source of embolism.

Specifically, if the strut 100 fractures in an early post-implantation stage, it may pull the adhesion portion 15 to generate displacement and deformation. Under the action of blood flow, a corresponding site may undergo uncontrolled movement and impact surrounding tissue. By disposing the limiting member 152, the strut 100 of the body 11 can be pulled and limited, thereby reducing the occurrence of such random movement and enhancing the protection effect on the tissue. For example, as illustrated in FIG. 1, if a fracture occurs at one or more connections between the second subportion 113 and the first subportion 111, the limiting member 152 (having one end connected to the first subportion 111 and the other end connected to the second subportion 113 or the adhesion portion 15) can prevent the second subportion 113 from detaching from the first subportion 111 as a result of the fracture. This maintains the overall structure of the implantable device 1 in an intended state and position, thereby avoiding uncontrolled movement of the fractured second subportion 113 near the implantation site that could otherwise injure the tissue.

In addition, referring to FIG. 5, if a local fracture occurs in the strut 100 of the second subportion 113, for example, at a location near the tip 101, two ends of the fracture site may undergo relative displacement and cause local structural deformation, posing a risk of tissue puncture. By providing the limiting member 152 with its two ends fixed to different locations on the strut 100 or the adhesion portion 15, the strut 100 on both sides of the fracture site can be pulled and limited. This keeps the two ends of the fracture site aligned and maintains a structural morphology substantially consistent with that in an unfractured state, thereby preventing the second subportion 113 from puncturing tissue due to displacement or structural alteration caused by the fracture.

Moreover, the limiting member 152 may limit detachment of large degradation fragments. During the degradation process of the second subportion 113, its original structure may change. In addition to normal release of degradation microparticles from the adhesion portion 15 into bloodstream, localized fragments may also detach and exist as free debris. Due to the relative flexibility of the adhesion portion 15, under the impact of blood flow-for example, when the adhesion portion 15 configured as a winding layer loses support at the fracture site of the wrapped second subportion 113, the winding thread may be locally stretched from its winding configuration and undergoes elongation. This causes the fractured second subportion 113 to protrude from the adhesion portion 15 and potentially cause tissue injury, or even to detach from the adhesion portion 15 and enter blood. Because the limiting member 152 has two ends relatively fixed and the length relatively unchanged, the limiting member can pull and limit the large degradation fragments generated by the second subportion 113. This reduces the phenomenon of such fragments detaching and entering the blood to cause thrombosis, and provides better protection for the surrounding tissue.

Specifically, the limiting member 152 may be of a linear or strip-shaped structure. One limiting member 152 may be disposed; and one end of the limiting member is connected to the first subportion 111, and the other end is connected to the second subportion 113 or the adhesion portion 15. Alternatively, the number of limiting members 152 may be at least two, and the limiting members 152 are respectively connected to different positions of the second subportion 113 and/or the adhesion portion 15. Thus, the fractured or detached second subportion 113 is more comprehensively limited through pull and limitation in different directions. In an example, referring to FIG. 1 and FIG. 5, in some embodiments, the second subportion 113 includes at least two struts 100, the two struts 100 are connected at a certain angle to form a structural morphology with crests and troughs, the adhesion portion 15 is attached to each strut 100, and each strut 100 and the adhesion portion 15 attached thereto are at least connected with a limiting member 152, to further reduce random movement of the detached fragments generated by fracture or degradation and enhance the use safety of the implantable device 1.

Optionally, referring to FIG. 1 and FIG. 5, two ends of the limiting member 152 are respectively connected to middles or positions near the middles of two struts 100, such that the limiting member 152 can achieve a better limiting effect.

Optionally, when at least two limiting members 152 are disposed, the limiting members 152 may be arranged in a crossed or spaced-apart manner.

For another example, the adhesion portion 15 may have a relatively high tensile strength, such that the adhesion portion 15 is less susceptible to fracture under the impact of blood flow in a living body, thereby reducing the introduction of large-particulate foreign matters into blood. The adhesion portion 15 can be easily broken by manual pulling, such that the adhesion portion 15 does not significantly impede implantation when a new implantable device 1 is implanted. Of course, the adhesion portion 15 can also be not easily broken by manual pulling,

For another example, the adhesion portion 15 can absorb a liquid and swell upon contact with a liquid, thereby further enhancing protective force of the adhesion portion 15 on living tissue. Of course, it may be understood that the adhesion portion 15 may not swell upon contact with the liquid.

For another example, the adhesion portion 15 may incorporate bioactive substances, such as anticoagulant drugs that promote endothelialization or prevent coagulation, or antiproliferative drugs with tissue growth-inhibiting properties.

Referring again to FIG. 1, the implantable device includes a leaflet assembly 12 and a skirt portion. The leaflet assembly 12 is fixed to the inner side of the body 11, and is preferably located on the inner side of the first subportion 111. The implantable device 1 in this example is specifically a prosthetic heart valve. The skirt portion includes an inner skirt (covered by an outer skirt 13 and not shown in FIG. 1) and/or the outer skirt 13, and the inner skirt covers an inner wall of the first subportion 111 and is configured to connect the leaflet assembly 12 to the body 11. The outer skirt 13 covers an outer surface of the first subportion 111, thereby increasing frictional force and enhancing a connecting effect between the implantable device 1 and living tissue; and the outer skirt 13 can also achieve the effect of preventing paravalvular leakage. The adhesion portion 15 of the prosthetic heart valve may be formed as a winding layer and may be sutured to the inner skirt or the outer skirt using the same suture material and a process similar to conventional suturing, thereby simplifying the preparation flow of the prosthetic heart valve. In addition, the adhesion portion 15 and the inner skirt may be connected into a whole, thereby improving the connection between the adhesion portion 15 and the inner skirt. The adhesion portion 15 may be wrapped around the second subportion 113 in a strip-like or sheet-like form. The inner skirt may also achieve an effect of a limiting member. Of course, the adhesion portion 15 and the inner skirt may also be disposed separately.

The prosthetic heart valve 1 may be a prosthetic pulmonary valve. After the prosthetic pulmonary valve is implanted into a human body, a valve frame of the prosthetic pulmonary valve is relatively prone to malapposition near an outflow end. The provision of the adhesion portion 15 can prevent thrombosis caused by residual frame fragments and tissue injury resulting from sharp fractured edges of such fragments. The prosthetic heart valve in this example may also be a prosthetic aortic valve, a prosthetic mitral valve, or a prosthetic tricuspid valve.

FIG. 8 to FIG. 11 are schematic diagrams of an implantable device according to some other embodiments of the present application. In the embodiments described below, the first subportion, the second subportion, and the adhesion portion included in each implantable device, as well as their interrelationships, specific structures, materials, and parameters, may refer to all or part of the features of the adhesion portion described in the foregoing implementation solutions, and correspondingly possess all or part of the beneficial effects thereof. Detailed description will not be repeated herein below.

FIG. 8 shows an implantable device 2, which is primarily applicable for situations where an implantation site is a branched lumen. A body of the implantable device 2 includes a first subportion 211 and a second subportion 213 connected to the first subportion, where the first subportion 211 is implanted against a wall of a relatively smaller branch vessel, while the second subportion 213 is covered with an adhesion portion (not marked). The second subportion 213 is partially suspended at an ostium of the branch vessel, i.e., a site connected to a main vessel.

FIG. 9 shows another implantable device 3, which is specifically a vascular stent and primarily applicable for situations where an implantation site is a main vessel having a branch vessel. A body of the implantable device 3 includes a first subportion 311 and a second subportion 313, where the first subportion 311 is implanted against a wall of the main vessel, the second subportion 213 is covered with an adhesion portion, and a part of the second subportion 313 directly faces an ostium of the branch vessel, i.e., a site connected to the main vessel.

FIG. 10 shows another implantable device 4, which is specifically a vena cava filter. The implantable device 4 is configured to be implanted into a vena cava. A body of the implantable device includes a first subportion 411 and a second subportion 413, the first subportion 411 abuts against an inner wall of the vena cava, and the second subportion 413 is suspended within the vena cava. The body of the vena cava filter includes a plurality of crossed filter struts, and the filter strut has a general shape similar to the stent strut described in the foregoing implementations, or may be formed by braiding metal wires. The filter strut or metal wire of the second subportion 413 is covered with an adhesion portion. The adhesion portion is attached to the suspended second subportion 413 in a manner of allowing blood flow to pass through hollowed-out portions or gaps of the body.

FIG. 11 shows yet another implantable device 5, which is specifically a covered stent. A body of the implantable device has an overall shape similar to that of a vascular stent and includes a first subportion 511 and a second subportion 513, where the first subportion 511 is provided with a covering membrane, the first subportion 511 together with the covering membrane is apposed to a vascular wall, a stent strut of the second subportion 513 is wrapped with an adhesion portion, and the second subportion 513 is partially or entirely suspended after implantation in a living body.

Referring to FIG. 12, the present application further provides a manufacturing method for an implantable device 1. The manufacturing method may include the following steps:
S1: select a base material for preparing the implantable device;
S2: process the base material to form a body of the implantable device, where the body includes a first subportion configured to be apposed to a wall of a lumen after the implantable device is implanted into the lumen, and a second subportion which is at least partially suspended after the implantable device is implanted into the lumen; and
S3: form an adhesion portion which is attached to the second subportion.

Specifically, the base material for preparing the implantable device is selected and processed, the body 11 of the implantable device 1 is prepared, and the prepared body 11 includes at least one strut 100. In one of examples, hollowed-out portions are formed among the struts 100. After the required body 11 is prepared, the adhesion portion 15 attached to the second subportion 113 is formed, and the adhesion portion 15 is at least disposed along the length direction of the strut 100, to cover at least a portion of an outer surface of the second subportion. After the adhesion portion 15 is disposed at a predefined position of the body 11 as required, the preparation of the implantable device 1 is completed.

Specifically, the base material may be a tube material, or a stent wire. The tube material or stent wire has different diameters, and the base material in a suitable size may be selected according to a specification of the implantable device to be manufactured. A specific material of the body 11, as well as a material and a forming method of the adhesion portion 15 may refer to relevant description of the implantable device 1 described above.

Specifically, when the body 11 of the implantable device 1 is prepared, the body 11 of a desired shape can be obtained by performing processes such as cutting and engraving on the tube material according to cutting requirements; alternatively, the body 11 can be obtained by weaving a stent wire according to a corresponding weaving method. In a possible embodiment, when the body 11 is prepared by a weaving method, in addition to configuring the adhesion portion 15 after the preparation of the body 11 is completed, the following may also be employed: during the weaving of the body 11, when the fabrication for a portion of the body 11 to which the adhesion portion 15 is to be configured is completed, the adhesion portion 15 may first be formed on the corresponding portion of the body 11, and thereafter the overall weaving of the body 11 is performed. In this way, the configuration of the adhesion portion 15 is facilitated. In addition, it is convenient to timely regulate the adhesion portion, thereby enhancing the accuracy and reliability of the adhesion portion 15.

Specifically, when the body 11 is prepared from a metal base material through processes such as drawing, engraving, and cutting, after the required body 11 is obtained by cutting a tube material with a cutting device, the obtained body 11 may first be subjected to optimization treatment, such as surface roughness treatment, to eliminate uneven structures such as burrs, pits, or protruding points at different positions, thereby achieving a required degree of surface finish for the stent. The surface roughness treatment may be mechanical grinding and polishing, and may also be chemical polishing. Moreover, the body 11 may also be subjected to heat treatment, to eliminate residual internal stress at different positions of the body 11, thereby enhancing the overall structural strength. During the heat treatment, the body 11 is placed into a heating furnace, slowly heated until approximately 400°C, then maintained at that temperature for 20-30 min, and cooled in the furnace. After the heat treatment of the body 11 is completed, subsequent processing may be performed, such as preparation or forming of the adhesion portion 15.

Specific implementations of the implantable device 1 in the present application are described in detail below with reference to examples and comparative examples.

### Example 1

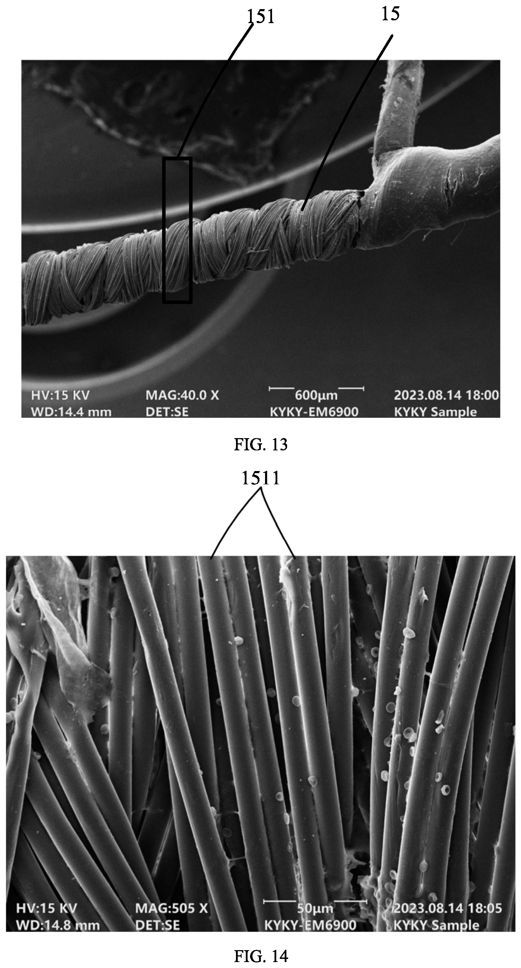

An implantable device 1 was an iron-based bioresorbable prosthetic pulmonary valve, and its structure is generally shown in FIG. 1. A main difference is that a hollowed-out portion included in the implantable device 1 of the example was quadrilateral instead of hexagonal as illustrated in FIG. 1, and an adhesion portion 15 was attached only to parts of struts 100 included in a second subportion 113. The adhesion portion 15 was a winding layer formed by winding a PLLA suture line, and covered some struts 100 of the suspended second subportion 113 at an outflow end. Referring together to FIG. 13 and FIG. 14, the adhesion portion 15 was formed by interweaving a winding thread 151 around the strut, and the winding thread 151 was formed by winding a plurality of turns of a PLLA suture bundle including a plurality of fibers 1511 in an overlapping pattern. The filament width of each turn was about 80-150 µm, and the diameter of the fibers was about 12 µm. The surface of the winding layer had irregular wavy undulations, with a peak-to-valley height of about 0.05-0.08 mm. The thickness of the winding layer was about 110 µm, the permeability coefficient was approximately 1×10⁻³ cm/s, and a procoagulation activity was about 20%. The leaflet frame material was a nitrided iron-based material, and the wall thickness of struts 100 was about 140 µm.

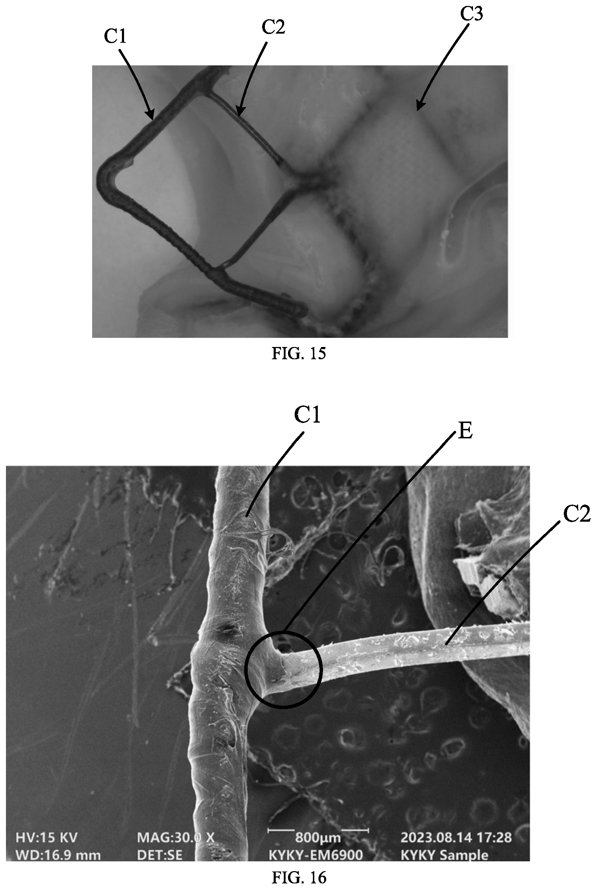

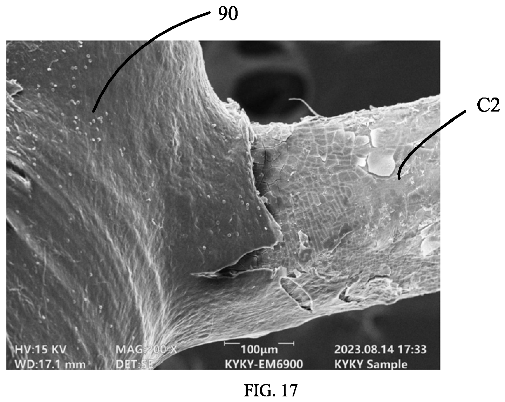

The implantable device 1 described above was implanted into an animal (dog, the same applies hereinafter). The animal survived for 30 days after implantation, and the device was then explanted. First, visual observation was conducted. As shown in FIG. 15, which is an image taken by an ordinary mobile phone, reference marks C1 and C2 in FIG. 15 indicated the stent struts that were suspended after implantation. The strut indicated by C1 was wrapped with the winding layer, while the strut indicated by C2 was a bare stent strut. C3 indicated an apposed portion of the implantable device 1. A translucent neointimal layer was formed at C1, and the degree of endothelialization was comparable to that at C3, whereas no obvious neointimal layer was observed at C2. The stent struts made of the iron-based material maintained original morphologies, without fractures and significant degradation, and the weight loss rate of the stent struts wrapped by the winding layer was about 2%. Local imaging of C1 and C2 was performed using a scanning electron microscope (SEM). As shown in FIG. 16 and FIG. 17, it can be observed that a neointimal layer with a coverage rate of nearly 100% was formed on the outer surface of the stent strut corresponding to C1. In contrast, only sporadic tissue was attached to the bare suspended stent strut at C2, with a neointimal coverage rate of less than 5%.

### Example 2

An implantable device 1 was an iron-based bioresorbable prosthetic pulmonary valve, and its structure shown in FIG. 1. The material of a stent frame and the material of a winding layer were the same as those in Example 1, and a difference is that the peak-to-valley height of the winding layer in Example 2 was about 0.08-0.12 mm, the thickness of the winding layer was about 130 µm, the permeability coefficient was about 1×10⁻³ cm/s, and the procoagulation activity was about 30%. The animal survived for 14 days after the implantable device was implanted in the animal. Upon retrieval of the device, it was observed that a substantially continuous and intact neointimal layer was formed on an outer surface of a second subportion 113 to which an adhesion portion 15 was attached, with the neointimal coverage rate greater than 90%, and the weight loss rate of a stent strut wrapped by a winding layer was approximately 1%.

### Example 3

An implantable device 1 was an iron-based bioresorbable prosthetic pulmonary valve. A difference from Example 2 is that the peak-to-valley height of a winding layer was about 0.03-0.05 mm, the thickness of the winding layer was about 110 µm, and the procoagulation activity was about 10%. The animal survived for 30 days after the implantable device was implanted in the animal. Upon retrieval of the device, it was observed that the neointimal coverage rate of an adhesion portion 15 was about 95%, and the weight loss rate of a stent strut wrapped by a winding layer was approximately 2%.

### Example 4

An implantable device 1 was an iron-based bioresorbable prosthetic pulmonary valve. A difference from Example 2 is that the peak-to-valley height of a winding layer was about 0.7 mm, the thickness of the winding layer was about 750 µm, and the procoagulation activity was about 90%. The animal survived for 30 days after the implantable device was implanted in the animal. Upon retrieval of the device, it was observed that the neointimal coverage rate of an adhesion portion 15 was 100%, and the weight loss rate of a stent strut wrapped by a winding layer was approximately 3%.

### Example 5

An implantable device 1 was an iron-based bioresorbable prosthetic pulmonary valve. A difference from Example 2 is that the peak-to-valley height of a winding layer was about 0.35 mm, the thickness of the winding layer was about 450 µm, and the procoagulation activity was about 50%. The animal survived for 30 days after the implantable device was implanted. Upon retrieval of the device, it was observed that the neointimal coverage rate of an adhesion portion 15 was 100%, and the weight loss rate of a stent strut wrapped by a winding layer was approximately 2%.

### Example 6

An implantable device 1 was an iron-based bioresorbable prosthetic pulmonary valve. A difference from Example 2 is that the filament width of each turn of a winding thread was about 500 µm, the spacing was 0.5 mm, and the procoagulation activity was about 10%. The animal survived for 30 days after the implantable device was implanted. Upon retrieval of the device, it was observed that the neointimal coverage rate of an adhesion portion 15 was about 70%, and the weight loss rate of a stent strut wrapped by a winding layer was approximately 1%.

### Examples 7-10

An implantable device 1 was an iron-based bioresorbable prosthetic pulmonary valve. Each example was approximately the same as Example 2, where in Examples 7-9, an adhesion portion 15 was formed by winding a PLLA suture line on a stent strut, and in Example 10, an adhesion portion 15 was a PLLA permeable membrane, the peak-to-valley height of the adhesion portion 15 in each example was 0.08-0.12 mm, and for main differences between each example and Example 2, see Table 1. 30 days, 90 days, and 1 year after the implantable device was implanted in an animal, the animal survived. Upon retrieval of the device at different time points, it was observed that the neointimal coverage rate of the adhesion portion 15 was 100%, and Table 1 shows the weight loss rate of the stent strut wrapped by a winding layer.

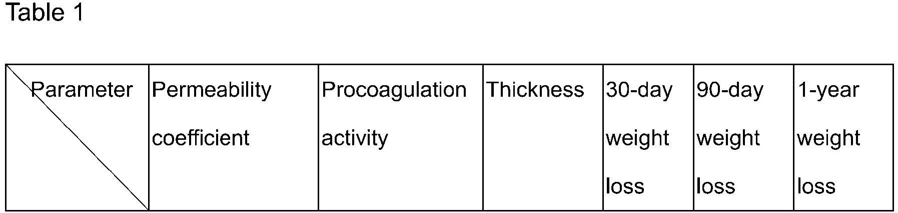

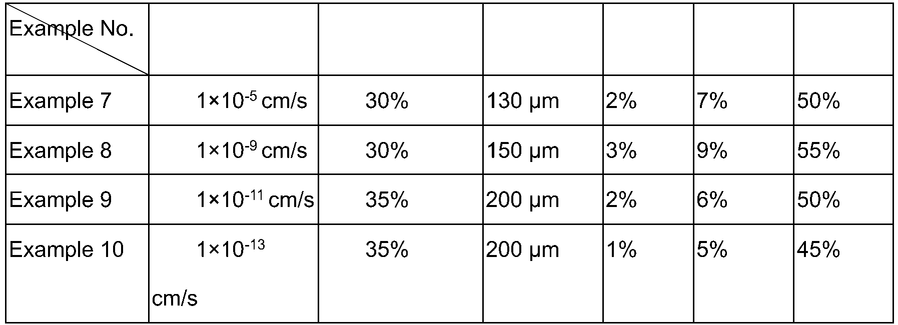

### Examples 11-14

An implantable device 1 was an iron-based bioresorbable prosthetic pulmonary valve. Each example was approximately the same as Example 2, an adhesion portion 15 was a polycaprolactone (PCL) spun fiber layer formed by a stent strut, the peak-to-valley height was 0.08-0.12 mm, and Table 2 shows main differences between each example and Example 2. 30 days, 90 days, and 1 year after the implantable device was implanted in an animal, the animal survived. Upon retrieval of the device at different time points, it was observed that the neointimal coverage rate of the adhesion portion 15 was 100%, and Table 2 shows the weight loss rate of the stent strut wrapped by a winding layer.

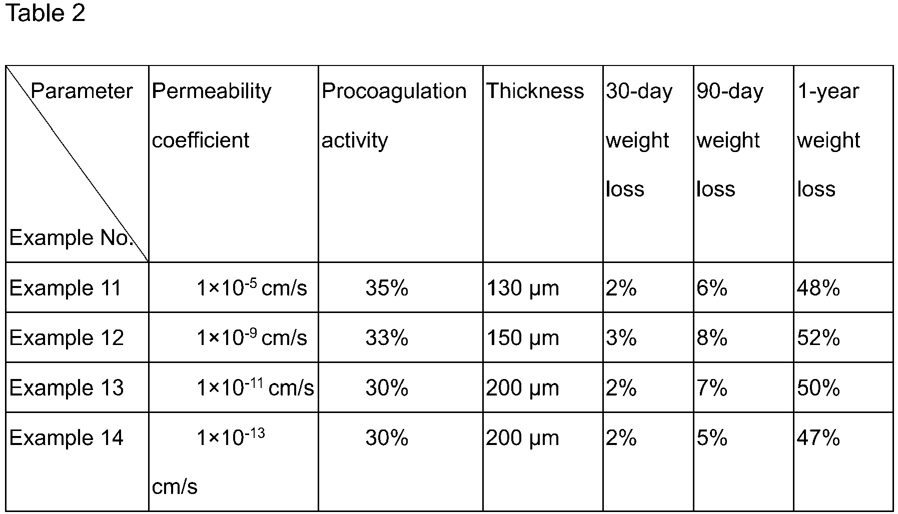

### Examples 15-17

An implantable device 1 was an iron-based bioresorbable prosthetic pulmonary valve, and its structure shown in FIG. 1. The wall thickness of a strut 100 was about 140 µm, an adhesion portion 15 wrapped all struts 100 included in a body 11, the adhesion portion 15 was made of PLLA, where the adhesion portion 15 in Example 15 was specifically a PLLA spun fiber layer, and the adhesion portion 15 in Examples 16-17 was a PLLA permeable membrane, the peak-to-valley height was 0.12-0.15 mm, and Table 3 shows other parameters in different examples. 30 days, 90 days, and 1 year after the implantable device was implanted in an animal, the animal survived. Upon retrieval of the device at different time points, it was observed that the neointimal coverage rate of the adhesion portion 15 was 100%, and Table 3 shows the weight loss rate of the stent strut wrapped by a winding layer.

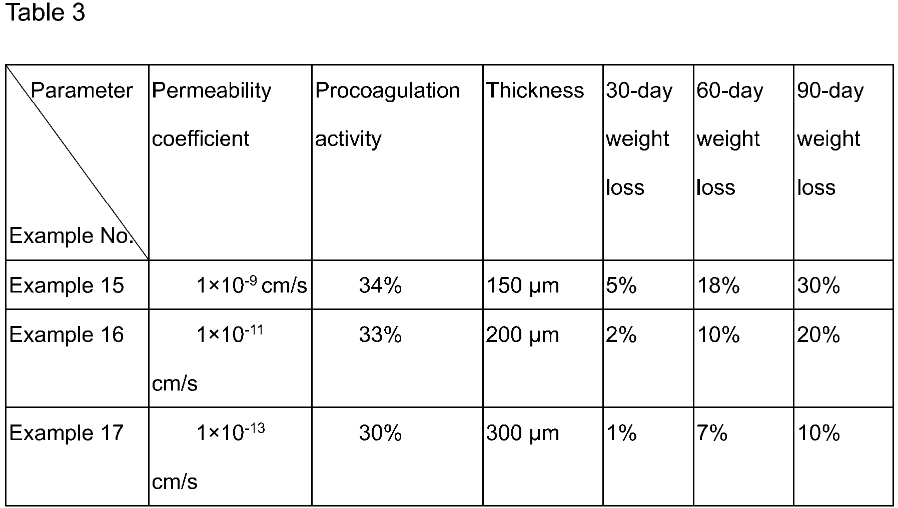

### Example 18

An implantable device 1 was a nitrided iron-based vena cava filter, a structure shown in FIG. 10 was adopted, and the wall thickness and the strut width of a filter strut were respectively 250 µm and 400 µm. A PLLA suture line was wound around all filter struts in a plurality of turns to form an adhesion portion, the filament width of a winding thread was about 50-100 µm, and the diameter of fibers was about 12 µm. The peak-to-valley height on a surface of a winding layer was about 0.03-0.05 mm. The thickness of the winding layer was about 130 µm, the permeability coefficient was approximately 1×10⁻³ cm/s, and the procoagulation activity was about 10%. The animal survived for 30 days after the implantable device was implanted. Upon retrieval of the device, it was observed that the neointimal coverage rate at suspended struts was 100%, suspended portions were not fractured, and the weight loss rate of the stent struts wrapped by the winding layer was approximately 3%.

### Example 19

An implantable device 1 was a zinc alloy vena cava filter, a structure shown in FIG. 10 was adopted, and the wall thickness and the strut width of a filter strut were respectively 250 µm and 400 µm. A PLLA suture line was wound around all filter struts in a plurality of turns to form an adhesion portion, the filament width of a winding thread was about 50-100 µm, and the diameter of fibers was about 12 µm. The peak-to-valley height on a surface of a winding layer was about 0.03-0.05 mm. The thickness of the winding layer was about 130 µm, the permeability coefficient was approximately 1×10⁻³ cm/s, and the procoagulation activity was about 10%. The animal survived for 30 days after the implantable device was implanted. Upon retrieval of the device, it was observed that the neointimal coverage rate at suspended struts was 98%, suspended portions were not fractured, and the weight loss rate of the stent struts wrapped by the winding layer was approximately 4%.

### Comparative Example 1

An implantable device 1 was an iron-based bioresorbable prosthetic pulmonary valve, and its structure shown in FIG. 1. The material and structure of a stent frame were the same as those in Example 1, and the wall thickness of the stent frame was 140 µm, an adhesion portion 15 was a PLLA permeable membrane, the peak-to-valley height on the surface of the adhesion portion 15 was about 0.01 mm, the thickness of the permeable membrane was 20 µm, the permeability coefficient was about 1×10⁻⁵ cm/s, and the procoagulation activity was about 5%. the animal survived for 30 days after the implantable device was implanted. Upon retrieval of the prosthetic pulmonary valve, it was observed that the neointimal layer was locally formed but discontinuous on a suspended second subportion 113 to which the adhesion portion 15 was attached, with the neointimal coverage rate below 30%, while the neointimal coverage rate of an apposed portion of a valve frame was 100%, and the weight loss rate of a stent strut wrapped by the permeable membrane was below 2%.

### Comparative Example 2

An implantable device 1 was an iron-based bioresorbable prosthetic pulmonary valve, and its structure shown in FIG. 1. The material and structure of a stent frame were the same as those in Example 1, and the wall thickness of the stent frame was 140 µm, an adhesion portion 15 was a PLLA permeable membrane, the peak-to-valley height on the surface of the adhesion portion 15 was about 0.8mm, the thickness of the permeable membrane was 850 µm, the permeability coefficient was about 1×10⁻⁴ cm/s, and the procoagulation activity was about 95%. After 30 days of implantation in an animal, the animal survived but exhibited signs of sluggish movement and lethargy. Upon retrieval of the prosthetic pulmonary valve, it was observed that thrombus was formed locally but had not yet detached from a suspended second subportion 113 to which the adhesion portion 15 was attached.

### Comparative Example 3

An implantable device 1 was a magnesium alloy bioresorbable prosthetic pulmonary valve. A valve frame was made of a magnesium alloy material and adopted a structure shown in FIG. 1, with a frame wall thickness of 140 µm. A suspended stent strut was a bare stent strut and not provided with an adhesion portion 15. At this moment, moisture can instantly reach the surface of the stent strut, equivalent to a permeability coefficient greater than 1×10⁻³ cm/s. After 30 days of implantation in an animal, the animal died. Necropsy and observation revealed that the suspended stent strut (i.e., a second subportion) at an outflow end of the valve frame was partially missing, and pulmonary embolism was found. Additionally, local separation occurred between a suspended portion and an apposed portion of the valve frame, and a leaflet assembly 12 was displaced downward relative to an original implantation site (corresponding to a native annulus). Only sporadic tissue was attached to the remaining suspended stent strut, without the formation of a continuous neointimal layer, and the neointimal coverage rate was below 5%.

The above descriptions are merely preferred specific embodiments of the present application, but the protection scope of the present application is not limited thereto. Any changes or substitutions that can be readily conceived by any person skilled in the art within the technical scope disclosed in the present application shall fall within the protection scope of the present application.

## Claims

1. An implantable device, comprising:
a body, wherein the body comprises a first subportion and a second subportion, the first subportion is configured to be apposed to a wall of a lumen after the implantable device is implanted into the lumen, the second subportion is connected to the first subportion; the second subportion is at least partially suspended after the implantable device is implanted into the lumen; and
an adhesion portion, wherein the adhesion portion is attached to the second subportion.

2. The implantable device of claim 1, wherein the surface of the adhesion portion has wavy undulations with a peak-to-valley height of 0.03-0.7 mm.

3. The implantable device of claim 1, wherein the adhesion portion has a permeability coefficient ranging from 1×10⁻¹³ to 1×10⁻³ cm/s.

4. The implantable device of claim 1, wherein the material of the adhesion portion exhibits a procoagulation activity of 10%-90%; and/or both the adhesion portion and the first subportion have negative surface potential polarity, and an absolute value of a surface potential of the adhesion portion is less than that of the first subportion.

5. The implantable device of claim 1, wherein the second subportion comprises at least one strut, the adhesion portion comprises at least one winding thread, the at least one winding thread is wound around the strut in a plurality of turns to form the adhesion portion.

6. The implantable device of claim 5, wherein the width or diameter of the winding thread is 50-700 µm; and/or a spacing between adjacent turns of the winding thread is 0-0.5 mm.

7. The implantable device of claim 6, wherein each winding thread comprises a plurality of fibers, the fibers have diameters ranging from 5 µm to 25 µm, which is uniform or varied.

8. The implantable device of claim 5, wherein the winding thread is wound around the strut in a manner selected from one or any combination of the following: overlapping winding, flat winding, multi-layered winding, variable-density winding; and/or an included angle between each turn of the winding thread and the strut axis is less than 90°; and/or an included angle between each turn of the winding thread and the strut axis varies along the axis.

9. The implantable device of claim 5, wherein the winding thread forms a plurality of binding knots on the strut; the binding knots are disposed adjacently or at intervals; and/or the adhesion portion further comprises a traction layer, the traction layer is disposed along the length direction of the strut of the second subportion, the winding thread and the traction layer form a plurality of intersection points; at each intersection point, the winding thread and the traction layer are connected or spaced apart to form an integrated structure.

10. The implantable device of claim 1, wherein the adhesion portion comprises a base layer and a plurality of fibers extending from the base layer; the base layer is wrapped around the second subportion; the fiber distribution density is 10-500 fibers/mm².

11. The implantable device of claim 1, wherein the adhesion portion has a thickness of 10-750 µm.

12. The implantable device of claim 11, wherein the second subportion comprises a tip; the adhesion portion has a thickness of 50-150 µm at the tip; and/or the second subportion comprises a fracture-prone region; the adhesion portion has a thickness of 20-100 µm in the fracture-prone region.

13. The implantable device of claim 1, wherein the adhesion portion is provided with a plurality of pores, each pore having a pore diameter of less than 500 µm; and/or the adhesion portion comprises a first density region and a second density region disposed adjacent to one another; the first density region is provided with a plurality of pores with a diameter ranging from 10 µm to 100 µm; the second density region is provided with a plurality of pores with a diameter ranging from 150 µm to 500 µm.

14. The implantable device of claim 1, wherein the shape of the adhesion portion is configured to conform to changes in the shape of the second subportion.

15. The implantable device of claim 1, further comprising:
a limiting member, wherein one end of the limiting member is connected to the first subportion, the other end of the limiting member is connected to one or both of the second subportion and the adhesion portion.

16. The implantable device of claim 15, wherein a plurality of limiting members are arranged; the limiting members are respectively connected to different positions on one or both of the second subportion and the adhesion portion.

17. The implantable device of claim 1, wherein the body or the second subportion is biodegradable; the degradation time of the second subportion is longer than the endothelialization time of the second subportion; and/or the adhesion portion is non-degradable; the degradation time of the adhesion portion is longer than the endothelialization time of the second subportion.

18. The implantable device of claim 1, further comprising a plurality of hollowed-out portions; wherein a flow channel is formed in the axial direction of the body; the second subportion is adjacent to at least one of the hollowed-out portions; the adhesion portion is attached to the second subportion in a manner that a fluid within the lumen can flow out from the flow channel through the hollowed-out portion adjacent to the second subportion.

19. The implantable device of any one of claims 1 to 18, further comprising:
a leaflet assembly, which is fixed to the inner side of the first subportion;
a skirt portion, which comprises at least one selected from the group consisting of an inner skirt and an outer skirt; wherein the inner skirt is configured to connect the leaflet assembly to the body; the outer skirt covers the outer side of the first subportion.

20. A manufacturing method for an implantable device, wherein the manufacturing method is configured to manufacture the implantable device of any one of claims 1 to 19, and comprises:
selecting a base material for preparing the implantable device;
processing the base material to form a body of the implantable device; wherein the body comprises a first subportion configured to be apposed to a wall of a lumen after the implantable device is implanted into the lumen, and a second subportion which is at least partially suspended after the implantable device is implanted into the lumen; and
forming an adhesion portion which is attached to the second subportion.
